Europäisches Patentamt

European Patent Office

Office européen des brevets

(19)

(11) Publication number: **0 140 681**
**A2**

# (12) EUROPEAN PATENT APPLICATION

(21) Application number: **84307378.4**

(22) Date of filing: **26.10.84**

(51) Int. Cl.⁴: **A 61 B 5/10**

(30) Priority: **28.10.83 JP 203337/83**

(43) Date of publication of application: **08.05.85**
**Bulletin 85/19**

(84) Designated Contracting States: **CH DE FR GB IT LI NL**

(71) Applicant: **Yoshizawa, Toru, 1-19-5, Shinmachi, Fuchu-shi Tokyo-to (JP)**

(72) Inventor: **Yoshizawa, Toru, 1-19-5, Shinmachi, Fuchu-shi Tokyo-to (JP)**
Inventor: **Okamoto, Yoshihisa, 20-173, Keyamyo Tarami-cho, Nishisonogi-gun Nagasaki-ken (JP)**

(74) Representative: **Harrison, David Christopher et al, MEWBURN ELLIS & CO 2/3 Cursitor Street, London EC4A 1BQ (GB)**

(54) **Examination for rachioscoliosis.**

(57) Disclosed is an apparatus of examining a patient in rachioscoliosis comprising means for: projecting a reference stripe-like pattern having a plurality of horizontal stripes on the back of the patient held by support means so as to form contour lines crossing the back; viewing the contour lines, which are distorted in dependence on the relief of the back of the patient, from an angle within the range between 1° and 90° with respect to the optical axis of the projection; measuring a difference between amounts of distortion on the right and left portions of the contour lines; and detecting any bilateral unsymmetricality on the basis of the measured difference.

EXAMINATION FOR RACHIOSCOLIOSIS

This invention relates to a method of examining a patient for rachioscoliosis or scoliosis and a device therefor.

It is well known that rachioscoliosis affects the health. For example, the lungs are oppressed so that the respiratory function may be lowered: in more serious cases, the ribs may be crazed. In addition, rachioscoliosis of course affects physical appearance. Since there are few subjective symptoms of rachioscoliosis, it is difficult to detect the condition in its early stages. If rachioscoliosis is detected in its early stages, it is possible to prevent its worsening by using a brace. However, when rachioscoliosis is not detected in its early stages, it is impossible to use the brace for treatment, and an operation becomes necessary. It is important therefore to find a reliable way of detecting rachioscoliosis in its early stages.

Since school-age children are specially liable to rachioscoliosis, it would be desirable to perform the detection in schools.

It is possible to directly detect rachio-

0140681

scoliosis by using a X-ray photography, but this method is undesirable for the reason that the irradiation of the X-ray should be avoided as much as possible from the medical point of view. In this connection, there is a demand for an excellent primary examination method by which rachioscoliosis can be easily and surely detected without using the X-ray photography. In this case, it is desirable that such a method can be used by people other than specialists such as orthopedists, for example, a teacher or a school doctor.

In order to examine schoolchildren in rachioscoliosis, an ocular inspection method has been most widely used. In this method, an inspector views the back of a patient who is bent forward. That is, he inspects whether there is an hump in the back of an patient and whether there is are elevations in back or loins, bilateral unsymmetricalness on the shoulders, shoulder-blades and the loins thereof. However, the ocular inspection is not reliable because it is obliged to rely on the inspector's subjectivity. Personal error is greatly varied from an inspector to another. The number of detected abnormalities is frequently dispersed over from several percent to several ten percent, and hence, this method is not considered to be objective one.

In order to cover the defect of the ocular inspection method, a primary examination method basd on a moire-topography has been recently developed and partially tried to use. In this method, a reference stripe-like pattern is projected on the back of a patient. The projected image is distorted in response to a relief of the back of the patient so as to produce a distorted stripe-pattern image of order of 2 - 3mm. By taking a photograph of the projected and distorted stripe-pattern image through the reference stripe-pattern, a moire-pattern in which the relief is illustrated by circular lines showing same height, is obtained. According to this method, degrees of two points of thus obtained moire-fringes are compared with each other, so that bilateral unsymmetricalness in the back of the patient, which are caused as the results of rachioscoliosis, can be detected. Hence, this method is more objective and more advantageous than the inspection method. However, it is not convenient to use the moire-topography method for the examination of schoolchildren because it is difficult to input data obtained from the moire-fringe to a computer necessary for processing a large amount of data and because it is necessary to rely on a skillful inspector for the reading of the moire-fringes. In addition, realization of the moire-topography method is expensive. Due to the above-described disadvantages, this method should not necessarily be the most suitable one as the primary examination method conducted in the schools.

Furthermore, in order to examine a patient for rachioscoliosis, in addition to the above two methods, there is a method using laser beam and a line sensor camera has been tried. In this examination method, the laser beams are horizontally irradiated to the back of the patient so as to form a bright spot thereon and then a position of the bright spot is detected by the line sensor camera which is disposed at a predetermined angle with the optical axis of the laser beams. By repeating this operation, it is possible to directly obtain data relative to contour lines crossing the back of the patient. However, much time has to be spent to obtain enough information necessary for the examination because it is necessary to move the line sensor camera and the laser beams around the patient. Also, there is a defect that the synchronism of all the data is not ensured because of breathing or the trembling of the patient. Accordingly, it is not expedient to use the laser beam method as a primary examination method.

E. Hierholzer, W. Frobin and others have tried to examine a patient for rachioscoliosis by projecting a stripe-like pattern on the back of the patient. In this method, the stripe-like pattern is projected on the back of the patient so as to form contour lines crossing the back, with the contour lines being distorted in response to a relief of the back. Since there is not used support means for securely holding the patient, the relief of the back of the patient is not emphasized so that patients having slight rachioscoliosis may be overlooked. Also, it is difficult to utilize a computer on line for processing data on the contour lines crossing the back because these contour lines are recorded by photographs. In addition, calibration on an optical system for the projection is complicated. Additionally, there remain such problems for practical use that the interpretation of the results is not uniformly made and tends to result in the confusion.

SUMMARY OF THE INVENTION

The inventors of the present invention have made their best efforts to obviate the above-described disadvantages of the various methods and achieved the present invention to provide an outstanding primary examination method of examining a patient for rachioscoliosis and a device therefor, wherein it is possible for everybody to perform the examination easily and rapidly and it is possible to detect securely a patient to be treated.

In accordance with this invention, a method of examining a patient for rachioscoliosis comprises the steps of: projecting a reference stripe-like pattern, or reference grating pattern, having a plurality of horizontal reference stripes on the back of the patient held by support means so as to form contour lines, or deformed grating image crossing the back by the projected holizontal stripes; viewing the contour lines, which are distorted in response to a relief of the patient, from an angle within the range between 1° and 90° with respect to the optical axis of the projection; measuring a difference between degrees of distortions on the right and left portions of the contour lines; and detecting a bilateral unsymmetricalness with respect to the contour lines on the basis of the measured difference.

Also, a device for examining a patient for rachioscoliosis comprises: support means for holding the patient in such a manner that he is forwardly inclined at an angle within the range from 5° and 20°; a projector spaced 0.5 - 20m apart from said support means to project the reference stripe-like pattern on the back of the patient so as to form contour lines crossing the back; means spaced substantially 0.5 - 20m from the support means for recording said contour lines which are distorted in response to a relief of the back of the patient; and means for detecting positions of the contour lines, and a rachioscoliosis examining system including a support post holding the projector and the means for recording.

BRIEF DESCRIPTION OF THE DRAWING

Figure 1 is an elevational view showing an embodiment of an examination device in accordance with this invention;

Figure 2 is a view showing an example of contour lines crossing the back of a patient;

Figure 3 is a view which illustrates a way of measuring a difference between degrees of distortions on the right and left portions of the contour lines as shown in Figure 2;

Figure 4 is a view of a reference stripe-like pattern for forming the contour lines of Figure 2;

Figure 5 is a block diagram showing an examination system in which data relative to the contour lines are directly inputted from a video camera to a computer;

Figure 6 is a block diagram showing another examination system in which data relative to the contour lines are inputted from a video camera to

0140681

a computer through a video tape; and

Figure 7 is a graphic chart showing an example of measured values of Example II.

DETAILED DESCRIPTION OF THE PREFERRED EMBODIMENTS

In the method according to this invention, a patient to be examined may stand upright, but it is preferable to incline the patient forwardly within the range between angles of 5° and 20° by using support means as mentioned later because it is possible to support the patient without trembling and because a relief of the back of a patient is emphasized by inclining the body of the patient so that even a slight rachioscoliosis can be detected. The reference stripe-like pattern can be projected on the back of a patient by using a conventional projector so as to form contour lines crossing the back, with the contour lines being distorted in response to the relief of the back. In this case, the projector has to be disposed so that the optical axis thereof is substantially perpendicular to the back of the patient. In order to symmetrically project the reference stripe-like pattern on the back of the patient as much as possible, it is preferable to perpendicularly intersect the optical axis of the projector with the longitudinal axis of the rachis at a center between the root of the neck and the loins of the patient. When a person stands

0140681

upright, the rachis is curved more or less. However, the term "longitudial axis of rachis" is herein defined as a vertical axis passing through the rachis of the patient who stands upright. Therefore, when the patient is inclined forwardly, the longitudinal axis of the rachis is also inclined at the same angle of inclination.

A distance between the back of the patient and the projector is changed depending upon a space between horizontal stripes of the reference stripe-like pattern to be projected and performance of the projector. The stripe-like pattern and the projector should be selected so that the distance between the back of the patient and the projector is within the range from 0.5 to 20m, preferably, 1 to 10m. For example, the stripe-like pattern can be obtained as a slide-film obtained by printing stripes on a film made of a transparent and not easily deformable material such as plastic or glass, and the pattern image is projected on the back of the patient. It is preferable from the data analysis by the computer that the reference stripe-like pattern includes a base stripe, a plurality of horizontal stripes which are disposed at regular intervals above and/or below the base stripe and which are parallel with the base stripe, and a vertical line which is intersected with the base stripe

0140681

and the horizontal stripes. In this case, it is preferable that the horizontal stripes are thinner than the base stripe. Especially, when the measurement is taken by the aid of a computer, it is advantageous to use the stripe-like pattern as mentioned above. A space between the adjacent horizontal stripes of the stripe-like pattern is selected so that, when it is projected on the back of the patient, a space between the projected horizontal stripes or the adjacent contour lines is within the range from 0.5 to 20cm, preferably 1 to 15cm, most preferably 3 to 10cm. If the space between the adjacent contour lines is too small, it becomes difficult to read the pattern image. Also, if it is too large, it is impossible to obtain necessary and enough data relative to the contour lines crossing the back. In order to facilitate positioning and setting the projector at a suitable angle, it is possible to form the vertical line of the reference stripe-like pattern as a solid line or a perforated line. Alternatively, the reference stripe-like pattern may have a mark showing the center of the projected image.

In the examination method according to this invention, the projected horizontal stripes or the contour lines, which are distorted in response to a relief of the back of a patient, may be viewed by suitable recording means such as a camera, a video camera or the like. In order to emphasize a degree of the distortion of the countor lines, it is necessary to view the contour lines from an angle within the range between 1° and 90°, preferably 10° and 60°, most preferably 20° and 50° with respect to the optical axis of the projector. That is, if the angle between the viewing axis and the optical axis of the projector is too small, it is impossible to emphasize much the distortion of the contour lines. On the contrary, if the angle is too large, it is difficult ot detect a degree of the distortion of the contour lines because these contour lines appear in a overlapping state.

In order to detect a bilateral unsymmetricalness on the countor lines crossing the back of the patient, that is, in order to examine whether the patient has rachioscoliosis, said bilateral unsymmetricalness is compared with that on the contour lines crossing the back of a normal person. In this case, it is preferable that the position and angle of the camera obscura or the video camera should be adjusted such that the viewing axis, along which the contour lines are viewed, must substantially exists in a plane which is defined by the optical axis of projection of the stripe-like pattern and the longitudinal axis of the rachis of the patient and should be intersected with the optical axis of the projection on the back, whereby the bilateral unsymmetricalness can be more strictly detected. It is apparent that the detection of the bilateral unsymmetricalness is performed by measuring a difference between degrees of distortions on the right and left portions of the contour lines crossing the back.

In the examination method according to this invention, it is possible to use a stripe-like pattern having vertical parallel stripes or a lattice-like pattern having horizontal and vertical stripes which are intersected with each other.

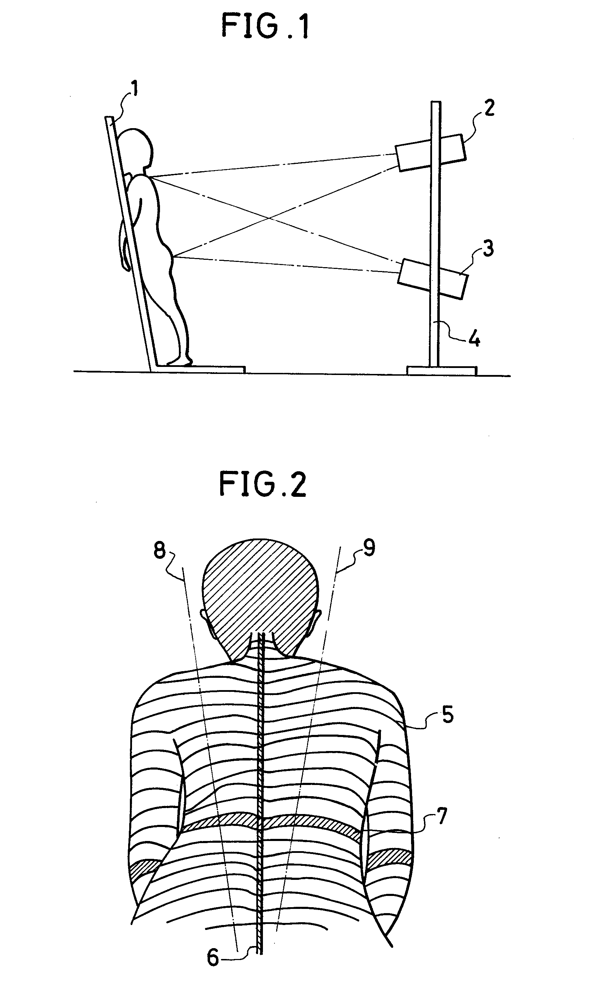

Referring to Figure 1, the examination device according to this invention comprises support means for holding a patient in such manner that he is forwardly inclined at an angle within the range from 5° to 20° so as to stabilize him, a projector 2 disposed to project the reference stripe-like pattern on the back of the patient held by the support means, a recorder 3 for viewing the projected and distorted stripe-like image which forms contour lines crossing the back of the patient, and a support device 4 for supporting the projector 2 and the recorder 3.

The support means for holding the patient can be suitably constructed on condition of securely fixing him. For example, it is possible to utilize a support device as used in an examination method based on a moire-topography. Such a support device comprises a base adapted to be secured on a floor and a plate member swingably mounted on the base. The plate member has a central opening which extends along the longitudinal axis thereof. After the patient to be examined stands upright on the base, the plate member is inclined at an angle within the range from 5° to 20° so that the shoulders and the hips of the patient are closely contacted with the plate member. Thus, trembling of the patient is completely prevented so that it is possible to obtain the contour lines which can be truly estimated. Preferably, the central opening of the plate member is spread out toward the top end thereof so that it can be fit for any one of patients having various breadths of the shoulders due to the age and the stature.

Instead of the plate member having the central opening, it is possible to use a plate member having two rods which are spaced apart from each other so that the head of the patient can be entered into the space between the rods. In this case, since better stabilization of the patient can be obtained while the relief of the back of the patient can be more clearly emphasized, it is possible to detect even the patient having slight rachioscoliosis.

As the projector, it is possible to utilize a slide-film projector of a conventional type. However, in this case, it is preferable to select the projector which has a powerful light source and lenses having small chromatic aberration. When the projector is used, the light rays for projection are not parallel so that a portion of the projected image which is apart from the optical axis is subjected to deformation. In order to eliminate this deformation as much as possible, it is preferable to adequately increase the distance between the projector and the back of the patient. If a computer is used to process data relative to the contour lines, it may be utilized to correct an error resulting from the deformation.

On the other hand, instead of the projector mentioned above, for example, it is possible to use a concave mirror which has a light source at its focus so as to obtain parallel light rays for using to project the stripe-like pattern on the back of the patient so as to form the comtour lines. Also, the contour lines may be directly formed on the back of the patient by using laser

0140681

beams and rotary scanning mirrors. According to these ways, the deformation problem as mentioned above can be solved. However, it is better to use the projector because realization of the two ways is expensive and because the deformation problem is not very serious. Accordingly, it is expedient to use the projector.

In this invention, the viewing of the contour lines crossing the back may be relied on an inspector. However, it is preferable to record the contour lines on a photograph or a video tape by using a camera obscura or a video camera due to efficiency of the examination, accuracy of the measurement and easiness in preserving of the records.

If a computer is utilized to process a large amount of data relative to the contour lines crossing the back, the video camera should be selected because it can be connected to the computer to input the data thereto.

Also, since the data relative to the contour lines crossing the back is suitable for digitization, it is possible to easily input the data to the computer. When a computer is used to process the data, it is preferable to use a solid-state imaging-sensing device camera as the video camera because it is possible to directly input the data to the computer.

The data relative to the contour lines may be inputted to the computer through the video tape.

As being apparent from the foregoing, it is possible to process the data online by using the video camera and the computer so that a high-speed processing of the data can be achieved. This is especially suitable for examining a great number of patients, for example, in a school.

As shown in Figure 1, the projector 2 and the camera can be supported by the same support post 4. It is preferable to dispose the projector 2 and the camera 3 on the support post at different levels in such a manner that a distance between the projector and the patient is near to a distance between the camera and the patient because it is unnecessary to take a difference between the distances into account. The viewing axis of the camera or the optical axis thereof has to substantially exist in a plane which is defined by the optical axis of the projector and the longitudinal axis of the rachis of the patient. In the arrangement shown in Figure 1, it is apparent that the projector 2 and the camera 3 can be easily adjusted so that the optical axis of the camera 3 is included in the plane mentioned above. Of course, each of the projector and the camera 3 may be supported on an individual support post.

Futhermore, it is possible to photograph the
contour lines crossing the back by reflecting it
in a mirror.  In addition, in order to emphasize
the contrast, the photograph should be made in a
dark room.

In Figure 2, the contour lines crossing the
back of the patient are shown by way of example.
As mentioned above, the contour lines, which are
indicated by the reference numeral 5, are formed
by pojecting the reference stripe-like pattern on
the back of the patient and are distorted in
response to the relief thereof.  When the contour
lines 5 are viewed by the camera, the distortion
of the contour lines 5 is suitably emphasized
depending upon the position of the means for
recording.

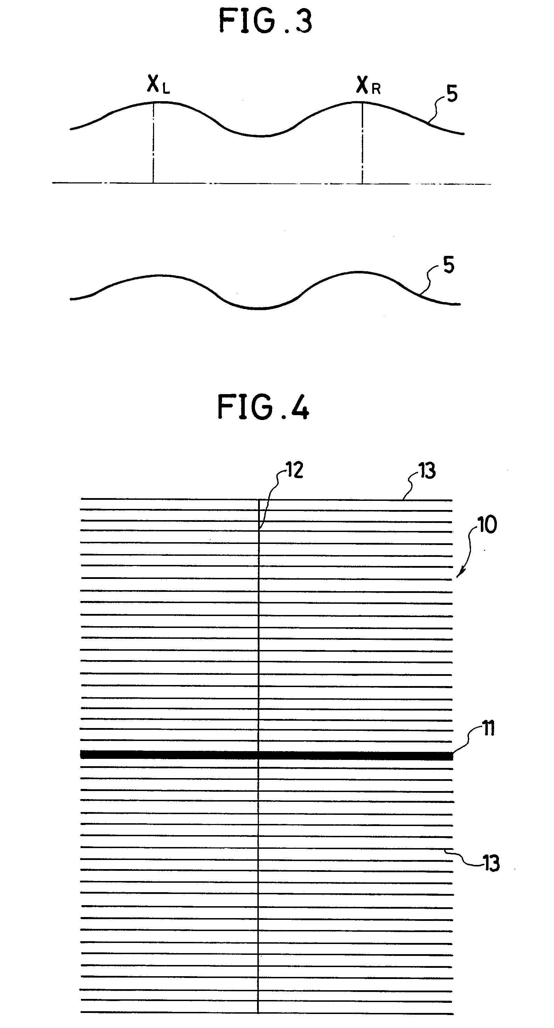

Figure 2 also shows two lines other than the
contour lines 5.  One of the two lines is
indicated by the reference numeral 6 and the other
is indicated by the reference numeral 7.  These
lines 6 and 7 are similarly obtained by projecting
the stripe-like pattern on the back of the
patient.  In Figure 4, the stripe-like pattern is
shown by way of example and is generally indicated
by the reference numeral 10.  As illustrated, the
reference stripe-like pattern 10 includes a base
stripe 11, a plurality of horizontal stripes 13
which are disposed at regular intervals above and below the base stripe 11 and which are parallel with the base stripe 11, and a vertical line 12 which is intersected at the right angle with the base stripe 11 and the horizontal stripes 13. The holizontal stripes 13 are thinner than the base stripe 11. On the contrary, the base stripe 11 may be thinner than the holizontal stripes, if necessary. It is, of course, apparent that the holizontal stripes 13, the vertical line 12 and the base stripe 11 respectively correspond to the contour lines 5, the lines 6 and 7.

In order to detect a bilateral unsymmetricalness with respect to the contour lines 5 for the examination of rachioscoliosis, it is necessary to measure a difference between degrees of distortions on the right and left portions of the contour lines 5. For example, when the contour lines 5 are recorded on a photograph, the measurement is performed by finding a difference between distances $X_L$ and $X_R$ as shown in Figure 3, which are gaugeable with a scale or the like. Figure 3 shows the adjacent contour lines 5 between which a straight line is drawn. The contour lines 5 are distorted in response to the relief of the back so that they are bilaterally curved as shown in Figure 3. The distances $X_L$ and $X_R$ respectively correspond to lengths of two line segments which are respectively drawn from peaks of the left and right curved portions to the horizontal line in such a manner that the line segments are perpendicular to the horizontal line.

Also, instead of using the distances $X_L$ and $X_R$, it is possible to utilize distances which are measured from the base line 7 to the peaks of the left and right curved portions of the corresponding contour line.

When the image data of the distorted stripe-pattern image are recorded on a recording medium of a computer, the difference is obtained by comparing the positional data of the heights of prominences at positions on one and the same contour measured from the reference line 7 and equidistant from the axis passing through the rachis by reading the coordinates on the computer.

When data relative to the contour lines 5 are inputted to a computer, the calculation as explained above is performed by the computer.

In order to elect points for measuring the difference between the degrees of the distortions on the right and left portions of the contour lines, it is expedient to use subsiduary lines 8 and 9 (Figure 2) which form a certain angle therebetween and generally make a right angle with the contour lines 5 because the back of a person narrows from the shoulders toward the loins. It is advantageous to measure the difference between the degrees of the distortions on the right and left portions of the contour lines at the points where the subsiduary lines 8 and 9 are intersected with the contour lines 5 because the measurement can be easily carried out in a short time and because the measured outcome has much correlation with the examination based on a X-ray photography. Accordingly, this manner is especially suitable for the examination on a great number of patients.

When the contour lines 5 are recorded on a video tape, the measurement may be performed by using a scale or the like because it is possible to reproduce an image of the recorded contour lines on a display device such as a TV set. That is to say, this is the same way as in the photograph on which the contour lines 5 are took. On the other hand, when data relative to the contour lines 5 are directly inputted from a video camera such as a solid-state image-sensing device camera to a computer, or when the data are inputted to the computer through the video tape, the measurement can be carried out by the computer. It is also possible to illustrate the recorded contour lines by inputting the data to a plotter such as a X-Y plotter so that the measurement can be performed by using a scale or the like. In this case, it is also expedient to utilized the subsidiary lines 8 and 9.

In this invention, it is necessary to use an external standard or an internal standard for converting the measured value into a true value. For this purpose, as the external standared, a primary standard having a given height, which is substituted for the patient, is previously recorded or photographed. On the other hand, as the internal standard, such a primary standard which is disposed along the body of the patient is simultaneously recorded or photographed together with the contour lines formed on the back thereof.

In the examination device according to this invention, in order to facilitate recording and various processing of a large amount of data, it is preferable to use a computer.

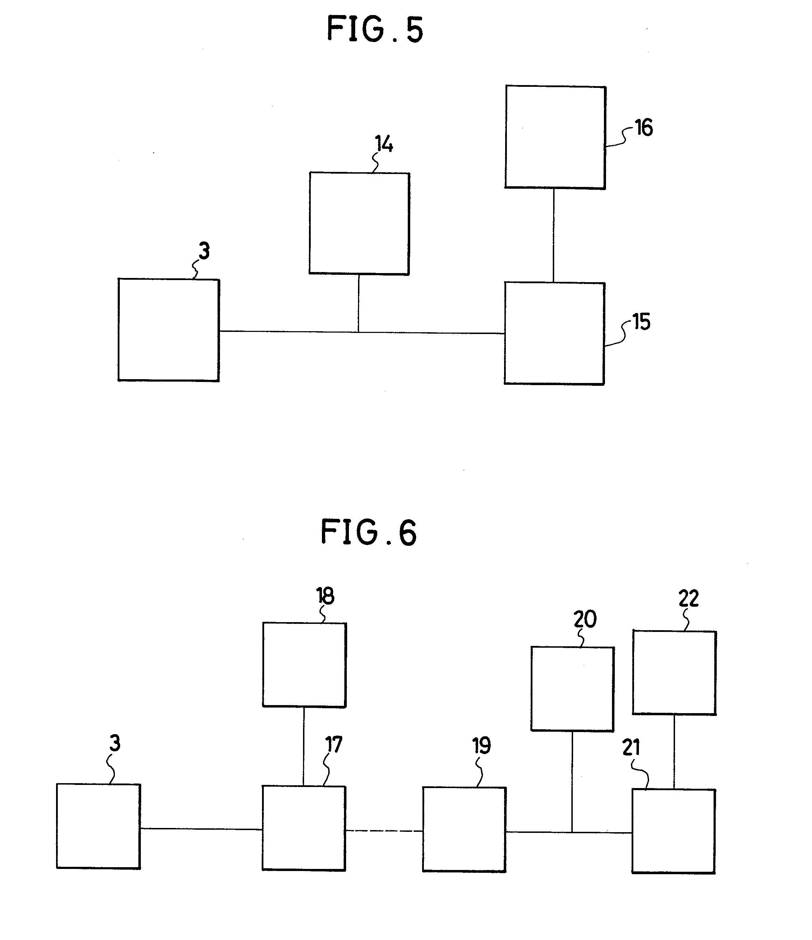

Figure 5 shows an embodiment of the examination system in which the data relative to the contour lines are directly inputted to a computer 15. In the arrangement shown in Figure 5, the output terminal of the recorder 3 is connected to the input terminals of a monitor 14 and the computer 15. The output terminal of the computer 15 is connected to the input terminal of a display device 16 such as a printer or the like. The contour lines formed on the back of the patient by projecting the reference stripe-like pattern from the projector are recorded by the recorder 3. The recorded information is inputted to the computer 15 which measures the difference between the degrees of the distortions on the right and left portions of the contour lines whereby ,the examination of rachioscoliosis is carried out.

Figure 6 shows another embodiment of the examination system in which the data relative to the contour lines are inputted to a computer 21 through a video tape. In the arrangement shown in Figure 6, the output terminal of the recorder 3 is connected to the input terminal of a video recorder 17 in which the inputted data are magnetically recorded. Then, the recorded data are fed to a monitor 18 to check operations of the recorder and the video recorder 17. The video tape in which the data are recorded is fed to a magnetic player and/or recorder 19. The output terminal of the magnetic player and/or recorder 19 is connected to the input terminals of a monitor 20 and the computer 21 and its output terminal is connected to the input terminal of a display device 21 such as a printer or the like.

As discussed above, by using the examination method and the examination device according to this invention, it is possible for everybody to examine easily and rapidly patients in rachioscoliosis. Also, it is easy to input the data relative to the contour lines to a computer so that the data can be processed by the computer. Therefore, this invention provides a method and a device which are suitable for examining a large number of patients in rachioscoliosis.

Now this invention will be further described in connection with the following examples:

Example I

The examination of rachioscoliosis was carried out by using a device which was constructed, as shown in Figure 1, from the following elements:

(a) support for patient, which is forwardly inclined at 8°;

(b) projector: focal distance 75mm lens 300w halogen lamp

(c) camera: 35mm single-lens reflex camera focal distance 55mm lens F11, exposure time 1/15sec. film tri-X ASA400

(d) scale graduated in millimeters First, preparations were made for examination as follows:

A patient was fixed on the support in a dark room. After a distance of 3m was set between the projector and the back of the patient, the projector was adjusted in such a manner that the optical axis of the projector was substantially perpendicular to the back of the patient and was substantially intersected with the longitudinal axis of the rachis at an angle of 90°, and that the center of the projection was positioned at an intermediate point between the root of the neck and the loins of the patient. In order to form contour lines crossing the back, a slide-film with a reference stripe-like pattern was prepared. The stripe-like pattern includes horizontal parallel stripes and a vertical line and the vertical line is intersected with the holizontal parallel strips. When the contour lines are formed on the back by projecting the reference stripe-like pattern, a space between the adjacent contour lines was about 5cm. Then, the camera was mounted on the same post as the projector. The camera was adjust in such a manner that the optical axis of the camera substantially existed in a plane which was defined by the optical axis of the projector and the longitudinal axis of the rachis, and that the optical axis of the camera was intersected with the optical axis at an angle 30° on the back of the patient.

For the examination, two persons were selected as a subject to be examined. That is, one person is normal and the other person has rachioscoliosis (Cobb angle of 20°) which was detected by a X-ray examination. Photographs of the contour lines of the two person were taken by using the device mentioned above. With respect to each of the photographs, a difference between degrees of the lateral distortion on one of the contour lines which is disposed in the vicinity of the thoracic vertebrae was measured by using a scale. Then, the measured value was converted into a real value by using a primary standard of 5cm photographed together with the contour lines. As a result of the conversion, it was found that the differences were about 3mm and 15mm on the normal person and the other person, respectively. This proves that a person having rachioscoliosis can be clearly distinguished from the normal person by the method according to this invention. In this example, it took about thirty seconds to photograph the contour lines and it took about forty seconds to measure the difference.

EXAMPLE II

The device used in EXAMPLE I was utilized, but the camera was replaced by a video camera. Twenty-two persons having rachioscoliosis were selected as a subject to be examined. The contour lines of all the persons were recorded on a video tape through which the data relative to the contour lines were inputted to a 16 bit personal computer. A difference between degrees of the bilateral destortion on the contour lines of each person was calculated by the computer. There is a favorable correlation between the given difference y (mm) and the Cobb angle x obtained from a X-ray examination, as shown in the following equation.

$$y = 1.02249x - 5.156 \quad (r(\text{correlation coefficient}) = 0.76)$$

It took about fifteen seconds to process the data per head.

The calculated values relative to one of the examined persons are shown in the following table I:

Table I

| Number of Contour Line | Measured Distances | | Difference |
| --- | --- | --- | --- |
| | Left | Right | |
| 6 | 5.0 | 3.0 | Left +2.0 |
| 5 | 12.0 | 9.0 | Left +3 |
| 4 | 19.5 | 16.0 | Left +3.5 |
| 3 | 27.0 | 24.0 | Left +3 |
| 2 | 34.5 | 31.5 | Left +3 |
| 1 | 41.5 | 39.5 | Left +2 |
| 0 | 53.0 | 52.5 | Left +0.5 |
| 1 | 65.5 | 65.5 | 0 |
| 2 | 73.5 | 74.0 | Left -0.5 |
| 3 | 81.0 | 81.0 | 0 |
| 4 | 88.5 | 88.0 | Left -0.5 |

*Contour Line Number 0:  Base Line

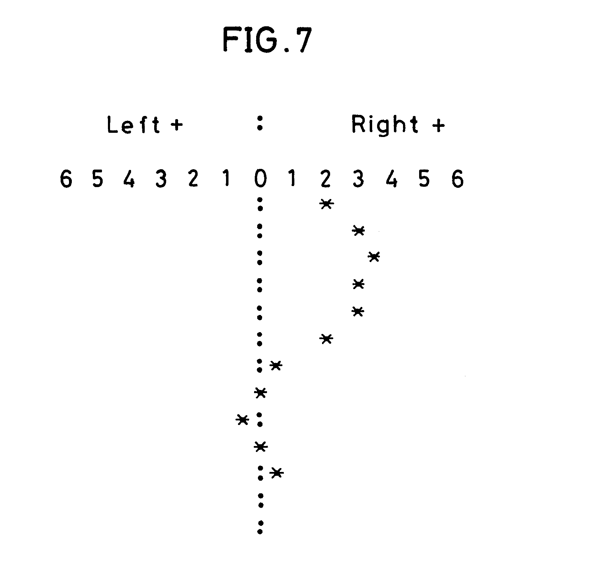

Also, these values are graphically represented in Figure 7.

Of course, in order for the examination of rachioscoliosis, it is possible to utilize table I and the graph of Figure 7.

EXAMPLE III

The system used in EXAMPLE II were utilized, but the data relative to the contour lines were directly inputted to the computer. Of course, the same outcome were obtained.

COMPARATIVE EXAMPLE

The subjects used in EXAMPLE I were examined by the moire-topography method. A difference of about 8mm between degrees of a lateral distortion on a moire was detected with respect to only the person having rachioscoliosis. In this method, it took about forty-five seconds to photograph the moire and it took even a skillful inspector about three minutes to read the moire.

**CLAIMS:**

1.     A device for examining a patient for rachioscoliosis comprising: support means for holding the patient in such a manner that he is forwardly inclined at an angle within the range from 5° to 20°, a projector spaced 0.5 - 20m apart from said support means to project a reference stripe-like pattern on the back of the patient so as to form contour lines crossing the back; means for recording said contour lines which are distorted in response to a relief of the back of the patient; and means for detecting positions of said contour lines.

2.     A device as set forth in Claim 1, wherein said support means include a pair of support members which are disposed apart from each other to hold the shoulder of the patient.

3.     A device as set forth in Claim 1 or Claim 2 wherein said projector and said recording means are supported by a common support post, the optical axis of said projector forms an angle within the range between 10° and 60° with the optical axis of said recording means.

4.     A device as set forth in any one of Claims 1 to 3, wherein said recording means includes a video camera, said detecting means includes a computor, the output terminal of said video camera is connected to the input terminal of said computer.

5. A device as set forth in any one of Claims 1 to 3, wherein said recording means include a video camera and a magnetic recorder associated therewith, said detecting means include a magnetic player and/or recorder and a computer associated therewith a computer, the playback output terminal of said magnetic player and/or recorder is connected to the input terminal of said computer.

6. A device as set forth in any one of Claims 1 to 5, wherein said reference stripe-like pattern includes a base stripe, a plurality of horizontal stripes which are disposed at regular intervals above and/or below said base stripe and which are parallel with said base stripe, and a vertical stripe which is intersected with said base stripe and said horizontal stripe, said horizontal stripes being thinner than said base stripe.

7. A method of examining a patient in rachioscoliosis comprising the steps of: projecting a reference stripe-like pattern having a plurality of horizontal stripes on the back of the patient held by support means so as to form contour lines crossing the back by the projected horizontal stripes; viewing the contour lines, which are distorted in response to a relief of the back of the patient, from an angle within the range between 1° and 90° with respect to the optical axis of the projection; measuring a difference between degrees of distortions on the right and left portions of the contour lines; and detecting a bilateral unsymmetricalness with respect to the contour lines on the basis of the measured difference.

8. A method as set forth in Claim 7, or device according to any one of Claims 1 to 6, wherein the optical axis of the projection is substantially perpendicular to the back of the patient, and spaces between the adjacent contour lines are within the range from 1 to 15cm.

9. A method as set forth in Claim 7 or Claim 8, or device according to any one of Claims 1 to 6, wherein a viewing axis along which the contour lines are viewed substantially exists in a plane which is defined by the optical axis of the projection and the longitudinal axis of the rachis, and is intersected with the optical axis of the projection on the back of the patient at an angle within the range between 10° and 60°.

# FIG.1

# FIG.2

0140681

# FIG.3

$X_L$ $X_R$ 5

5

# FIG.4

12 13

10

11

13

3/4

0140681

# FIG.5

# FIG.6

0140681

4/4

# FIG.7

Left +         :         Right +

6 5 4 3 2 1 0 1 2 3 4 5 6